# EUROPEAN PATENT APPLICATION

(11) **EP 3 542 801 A1**
(43) Date of publication of application: **25.09.2019**
(21) Application number: 19169300.1
(22) Date of filing: 15.08.2008
(51) Int. Cl.: A61K 31/5025, A61K 31/517, A61K 31/522, A61K 31/55, A61K 31/5513, A61K 31/553, A61P 35/00

(54) **PURIN DERIVATIVES FOR USE IN THE TREATMENT OF FAP-RELATED DISEASES**

(30) Priority: 17.08.2007 EP 07114494
(62) Divisional of application: 08787268.5
(71) Applicant: Boehringer Ingelheim International GmbH, 55216 Ingelheim am Rhein (DE)
(72) Inventor: Himmelsbach, Frank, 55216 INGELHEIM AM RHEIN (DE); Mark, Michael, 55216 INGELHEIM AM RHEIN (DE); Tadayyon, Mohammad, 55216 INGELHEIM AM RHEIN (DE); Thomas, Leo, 55216 INGELHEIM AM RHEIN (DE)
(74) Representative: Simon, Elke Anna Maria

(57) **Abstract**

The specification describes the use of selected purine derivatives for the treatment of hyperproliferative diseases.

## Description

The specification describes the use of substances that have inhibitory capabilities, for the treatment of all kinds of pathological conditions.

Fibroblast Activation Protein (FAP), also known as seprase, belongs to the family of serine proteases. FAP, like the enzyme DPP IV (dipeptidylpeptidase IV), for example, belongs to the enzymes that cleave a didpeptide from an existing protein or peptide with the general formula X-Pro-XAA. FAP is a transmembrane protein consisting of 760 amino acids. FAP exhibits a high homology with DPP IV and also forms heterodimers with this protein. Unlike DPP IV, the expression and activity of FAP is very limited. Thus, FAP is not expressed in normal adult tissues. FAP is induced in activated fibroblasts after trauma or injury to the tissue. FAP is also expressed in tumour stroma tissue of all kinds of human epithelial tumours and in malignant cells of various bone and soft tissue sarcomas. These include, *inter alia*, more than 90% of breast, non-small-cell lung and colorectal carcinomas. FAP is preferably found here in fibroblasts that occur close to newly forming or formed blood vessels and form a specific cellular compartment between the tumour capillary endothelium and the actual malignant epithelial cells and clusters of cells. FAP-positive fibroblasts are found both in primary carcinomas and in metastasising carcinomas. The expression profile of FAP suggests that FAP plays a part in tumour invasion into healthy tissue and in tumour formation and metastasis.

FAP inhibitors, i.e. substances that are capable of reducing or inhibiting the proteolytic activity of FAP, are useful therapeutic agents for the treatment of all kinds of tumour diseases. FAP inhibitors can preferably be used to treat tumours of epithelial origin such as breast tumours, non-small-cell lung carcinomas, colorectal carcinomas and soft tissue carcinomas. FAP inhibitors are also indicated in all kinds of metastasising tumours such as melanomas, for example.

In addition, FAP inhibitors are also indicated in other hyperproliferative diseases. These include, *inter alia*, cardiac hypertrophy, cirrhoses and fibromatoses. Moreover, FAP inhibitors may also be used as valuable therapeutic agents for treating rheumatic spectrum diseases such as e.g. arthritis or osteoarthritis and neurotraumatic disorders.

FAP inhibitors are also indicated for treating wound healing disorders and acne and proliferative skin complaints such as psoriasis, for example.

Additionally, FAP inhibitors are indicated for treating pain of all origins and migraine.

Selected purine derivatives according to the present invention may be defined by the formula (I), 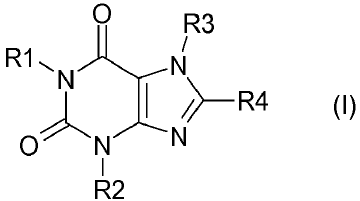 formula (II), 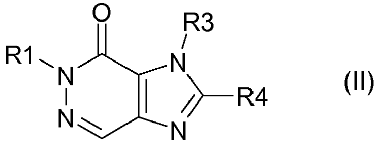 formula (III), 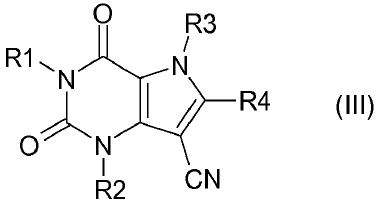 or formula (IV), 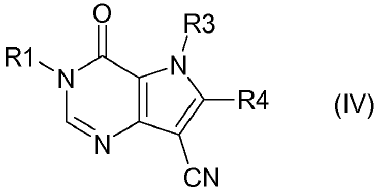 wherein
**R1** denotes pyridinylmethyl, pyrimidinylmethyl, quinolinylmethyl, (2-oxo-1,2-dihydro-quinolinyl)methyl, isoquinolinylmethyl, quinazolinylmethyl, (4-oxo-3,4-dihydro-quinazolinyl)methyl, quinoxalinylmethyl, [1,5]naphthyridinylmethyl, (1H-perimidinyl)methyl, phenanthridinylmethyl, (11H-dibenzo[b,e]azepinyl)methyl, (dibenzo[b,f][1,4]oxazepinyl)methyl, (5H-dibenzo[b,e][1,4]diazepinyl)methyl or (imidazo[1,2-a]quinolinyl)methyl and the heterocyclic groups of the above-mentioned groups may be mono- or disubstituted by Ra, while the substituents may be identical or different and **Ra** denotes fluorine, chlorine, bromine, cyano, methyl, ethyl, propyl, isopropyl, cyclopropyl, phenyl, methoxy, ethyloxy, amino, methylamino, dimethylamino, pyrrolidino, piperidino, morpholino, piperazino or N-methylpiperazino,
**R2** denotes methyl, ethyl, propyl, isopropyl, cyclopropyl or phenyl and the methyl, ethyl, propyl and isopropyl group may be substituted by carboxy, methoxycarbonyl, ethyloxycarbonyl, aminocarbonyl, methylaminocarbonyl, dimethylaminocarbonyl, pyrrolidinocarbonyl, piperidinocarbonyl, morpholinocarbonyl, piperazinocarbonyl or N-methylpiperazinocarbonyl,
**R3** denotes 2-buten-1-yl, 3-methyl-2-buten-1-yl, 2-butyn-1-yl, benzyl, fluorobenzyl, chlorobenzyl, bromobenzyl or cyanobenzyl and
**R4** denotes piperazino, homopiperazino, 3-(R)-amino-piperidin-1-yl, 3-(S)-amino-piperidin-1-yl, (2-amino-ethyl)-methylamino, (2-amino-2-methyl-propyl)-methylamino, ((R)-2-amino-propyl)-methylamino or ((S)-2-amino-propyl)-methylamino,
the tautomers, enantiomers and salts thereof, and mixtures thereof.

Of the purine derivatives of formulae (I) to (IV) those of formulae (I) and (II) are particularly preferred.

Preferred meanings of **R1** are pyridin-2-ylmethyl, pyridin-3-ylmethyl, pyridin-4-ylmethyl, pyrimidin-2-ylmethyl, pyrimidin-4-ylmethyl, quinolin-2-ylmethyl, quinolin-3-ylmethyl, quinolin-4-ylmethyl, quinolin-5-ylmethyl, quinolin-6-ylmethyl, quinolin-7-ylmethyl, quinolin-8-ylmethyl, (2-oxo-1,2-dihydro-quinolin-6-yl)methyl, isoquinolin-1-ylmethyl, isoquinolin-3-ylmethyl, isoquinolin-4-ylmethyl, isoquinolin-8-ylmethyl, quinazolin-2-ylmethyl, quinazolin-4-ylmethyl, quinazolin-6-ylmethyl, quinazolin-7-ylmethyl, (4-oxo-3,4-dihydro-quinazolin-2-yl)methyl, quinoxalin-2-ylmethyl, quinoxalin-5-ylmethyl, quinoxalin-6-ylmethyl, [1,5]naphthyridin-2-ylmethyl, [1,5]naphthyridin-3-ylmethyl, [1,5]naphthyridin-4-ylmethyl, (1H-perimidin-2-yl)methyl, phenanthridin-6-ylmethyl, (11H-dibenzo[b,e]azepin-6-yl)methyl, (dibenzo[b,f][1,4]oxazepin-11-yl)methyl, (5H-dibenzo[b,e][1,4]diazepin-11-yl)methyl or (imidazo[1,2-a]quinolin-2-yl)methyl, while the heterocyclic groups of the above-mentioned groups may be monosubstituted by cyano, ethyl, cyclopropyl, phenyl or morpholino or mono- or disubstituted by methyl,

particularly 3-cyano-pyridin-2-ylmethyl, (4-phenyl-pyrimidin-2-yl)methyl, (4,6-dimethyl-pyrimidin-2-yl)methyl, 3-cyano-quinolin-2-ylmethyl, 3-methyl-isoquinolin-1-ylmethyl, 4-cyano-isoquinolin-3-ylmethyl, quinazolin-2-ylmethyl, (1-methyl-2-oxo-1,2-dihydro-quinolin-6-yl)methyl, (4-methyl-quinazolin-2-yl)methyl, (4,5-dimethyl-quinazolin-2-yl)methyl, (4-ethyl-quinazolin-2-yl)methyl, (4-cyclopropyl-quinazolin-2-yl)methyl, (4-cyano-quinazolin-2-yl)methyl, (4-morpholino-quinazolin-2-yl)methyl, (4-phenyl-quinazolin-2-yl)methyl, (4-oxo-3,4-dihydro-quinazolin-2-yl)methyl, quinoxalin-2-ylmethyl, quinoxalin-6-ylmethyl, [1,5]naphthyridin-2-ylmethyl, (1H-perimidin-2-yl)methyl, phenanthridin-6-ylmethyl, (11H-dibenzo[b,e]azepin-6-yl)methyl, (dibenzo[b,f][1,4]oxazepin-11-yl)methyl, (5-methyl-5H-dibenzo[b,e][1,4]diazepin-11-yl)methyl or (imidazo[1,2-a]quinolin-2-yl)methyl.

Preferred meanings of **R2** are methyl, carboxymethyl, methoxycarbonylmethyl, ethyloxycarbonylmethyl, cyclopropyl and phenyl,
particularly methyl, carboxymethyl and phenyl.

Preferred meanings of **R3** are 2-buten-1-yl, 3-methyl-2-buten-1-yl, 2-butyn-1-yl, benzyl, 2-chlorobenzyl, 2-bromobenzyl or 2-cyanobenzyl, particularly 3-methyl-2-buten-1-yl, 2-butyn-1-yl or benzyl.

Preferred meanings of **R4** are piperazino, homopiperazino, 3-(*R*)-amino-piperidin-1-yl, 3-(*S*)-amino-piperidin-1-yl, (2-amino-ethyl)-methylamino, ((*R*)-2-amino-propyl)-methylamino or ((*S*)-2-amino-propyl)-methylamino.

The purine derivatives of general formulae (I) to (IV) can be prepared using methods known from the literature. The purine derivatives of general formula (I) can be prepared for example as described in WO 2002/068420, WO 2004/018468, WO 2004/041820, WO 2005/051950, WO 2005/082906, WO 2005/085246, WO 2006/027204 and WO 2006/029769.

The purine derivatives of general formula (II) can be prepared for example as described in WO 2004/050658, WO 2004/111051, WO 2005/058901, WO 2005/097798 and WO 2005/110999.

The purine derivatives of general formulae (III) and (IV) can be prepared for example as described in WO 2006/068163 and WO 2007/071738.

Particularly preferred purine derivatives are the following compounds, the tautomers, enantiomers and the therapeutically effective salts thereof:
- 1-[(quinazolin-2-yl)methyl]-3-methyl-7-(2-butyn-1-yl)-8-((*R*)-3-amino-piperidin-1-yl)-xanthine (cf. WO 2004/018468, Example 2(80)): 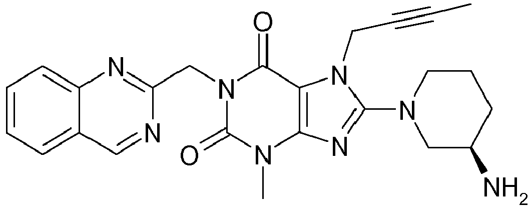
- 1-[(3-methyl-isoquinolin-1-yl)methyl]-3-methyl-7-(2-butyn-1-yl)-8-((*R*)-3-amino-piperidin-1-yl)-xanthine (cf. WO 2004/018468, Example 2(130)): 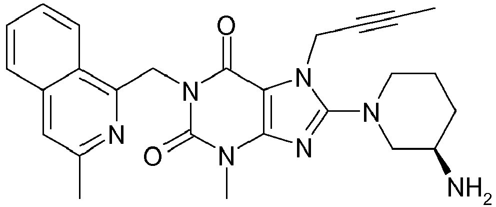
- 1-[(4-methyl-quinazolin-2-yl)methyl]-3-methyl-7-(2-butyn-1-yl)-8-((*S*)-3-amino-piperidin-1-yl)-xanthine (cf. WO 2004/018468, Example 2(141)): 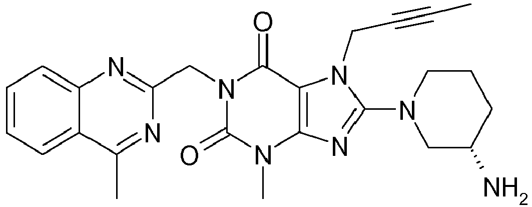
- 1-[(4-methyl-quinazolin-2-yl)methyl]-3-methyl-7-(2-butyn-1-yl)-8-(3-(*R*)-amino-piperidin-1-yl)-xanthine (cf. WO 2004/018468, Example 2(142)): 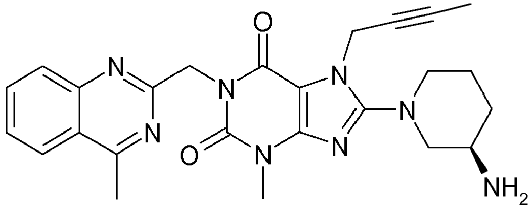
- 1-[(4-phenyl-quinazolin-2-yl)methyl]-3-methyl-7-(2-butyn-1-yl)-8-(3-(*R*)-amino-piperidin-1-yl)-xanthine (cf. WO 2004/018468, Example 2(217)): 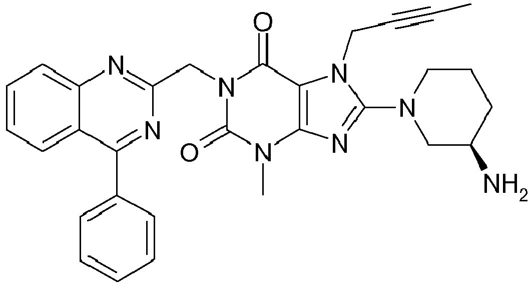
- 2-((R)-3-amino-piperidin-1-yl)-3-(but-2-ynyl)-5-(4-methyl-quinazolin-2-ylmethyl)-3.5-dihydro-imidazo[4,5-d]pyridazin-4-one (cf. WO 2004/050658, Example 136)): 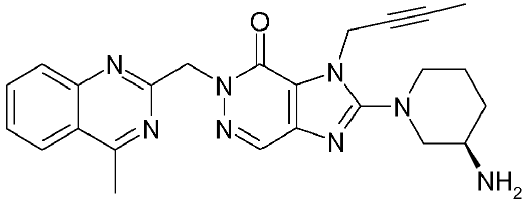
- 1-[([1,5]naphthyridin-2-yl)methyl]-3-methyl-7-(2-butyn-1-yl)-8-((*R*)-3-amino-piperidin-1-yl)-xanthine (cf. WO 2004/018468, Example 2(252)): 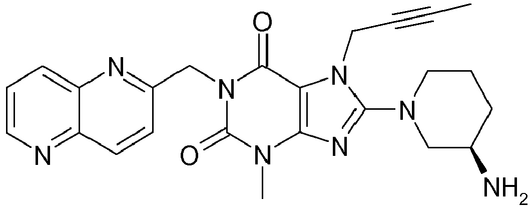
- 1-[(1H-perimidin-2-yl)methyl]-3-methyl-7-(2-butyn-1-yl)-8-(3-(*R*)-amino-piperidin-1-yl)-xanthine (cf. WO 2004/041820, Example 1(29)): 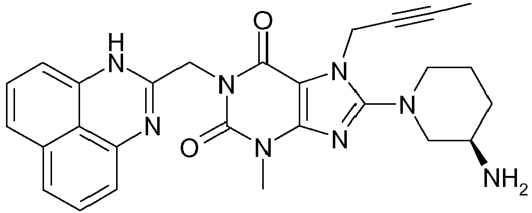
- 1-[(4-ethyl-quinazolin-2-yl)methyl]-3-methyl-7-(2-butyn-1-yl)-8-(3-(*R*)-amino-piperidin-1-yl)-xanthine (cf. WO 2005/085246, Example 1(22)): 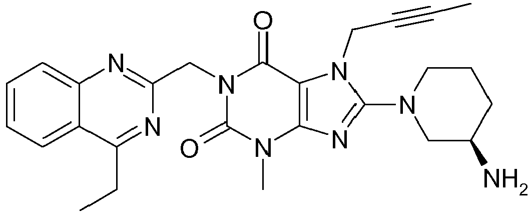
- 1-[(4-cyclopropyl-quinazolin-2-yl)methyl]-3-methyl-7-(2-butyn-1-yl)-8-(3-(*R*)-amino-piperidin-1-yl)-xanthine (cf. WO 2005/085246, Example 1(23)): 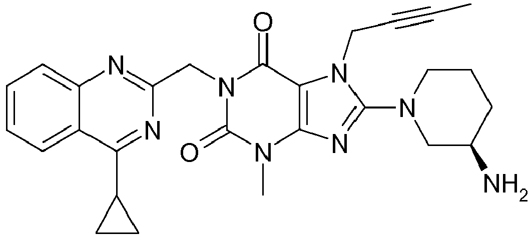
- 1-[(4-phenyl-pyrimidin-2-yl)methyl]-3-methyl-7-(2-butyn-1-yl)-8-(3-(*R*)-amino-piperidin-1-yl)-xanthine (cf. WO 2005/085246, Example 1(31)): 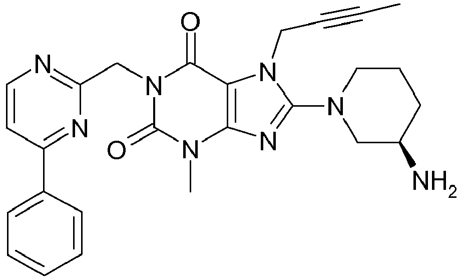
- 1-[(4-cyano-quinazolin-2-yl)methyl]-3-methyl-7-(2-butyn-1-yl)-8-(3-(*R*)-amino-piperidin-1-yl)-xanthine (cf. WO 2005/085246, Example 1(37)): 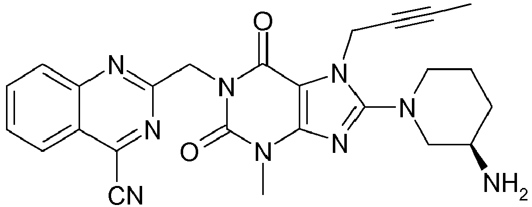
- 1-[(11H-dibenzo[b,e]azepin-6-yl)methyl]-3-methyl-7-(2-butyn-1-yl)-8-(3-amino-piperidin-1-yl)-xanthine (cf. WO 2004/041820, Example 1(5)): 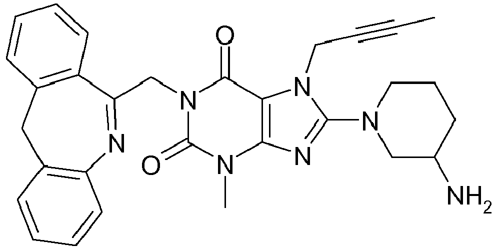
- 1-[(phenanthridin-6-yl)methyl]-3-methyl-7-(2-butyn-1-yl)-8-(3-(*R*)-amino-piperidin-1-yl)-xanthine (cf. WO 2004/041820, Example 1(33)): 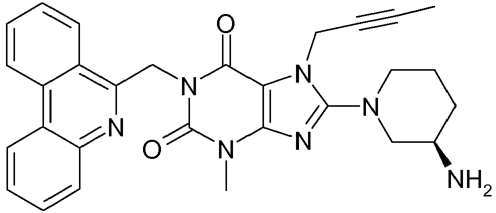
- 1-[(dibenzo[b,f][1,4]oxazepin-11-yl)methyl]-3-methyl-7-(2-butyn-1-yl)-8-(3-(*R*)-amino-piperidin-1-yl)-xanthine (cf. WO 2004/041820, Example 1(8)): 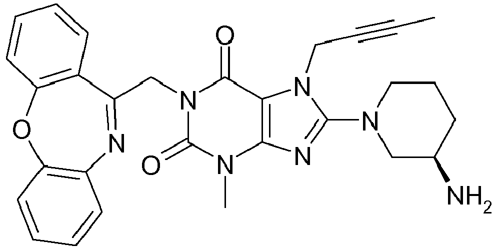
- 1-[(5-methyl-5H-dibenzo[b,e][1,4]diazepin-11-yl)methyl]-3-methyl-7-(2-butyn-1-yl)-8-(3-amino-piperidin-1-yl)-xanthine (cf. WO 2004/041820, Example 1(12)): 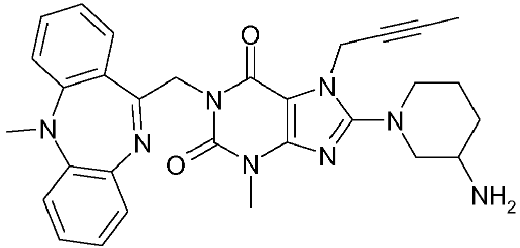
- 1-[(4,5-dimethyl-quinazolin-2-yl)methyl]-3-methyl-7-(2-butyn-1-yl)-8-(3-(*R*)-amino-piperidin-1-yl)-xanthine (cf. WO 2005/085246, Example 1(28)): 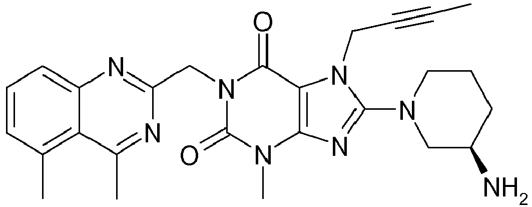
- 1-[(4-methyl-quinazolin-2-yl)methyl]-3-methyl-7-(2-butyn-1-yl)-8-[(2-amino-ethyl)-methylamino]-xanthine (cf. WO 2006/029769, Example 1(1)): 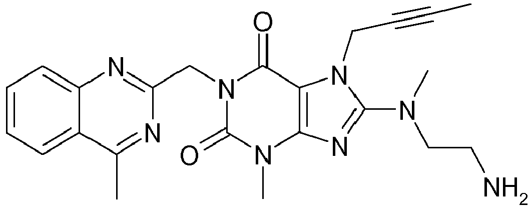
- 1-[(4-methyl-quinazolin-2-yl)methyl]-3-phenyl-7-(2-butyn-1-yl)-8-(3-(*R*)-amino-piperidin-1-yl)-xanthine (cf. WO 2005/082906, Example 1(8)): 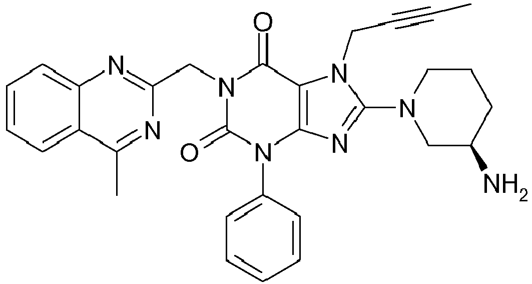
- 1-[(4-methyl-quinazolin-2-yl)methyl]-3-methyl-7-(3-methyl-2-buten-1-yl)-8-(3-(R)-amino-piperidin-1-yl)-xanthine (cf. WO 2004/018468, analogously to Example 2(20)): 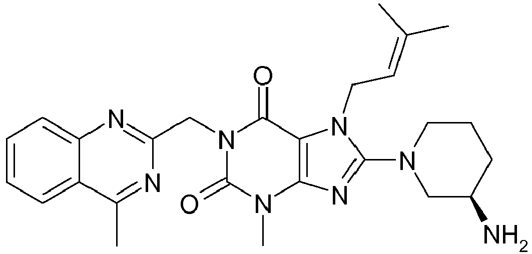
- 1-[(4-methyl-quinazolin-2-yl)methyl]-3-methyl-7-benzyl-8-(3-(*R*)-amino-piperidin-1-yl)-xanthine (cf. WO 2004/018468, analogously to Example 2(294)): 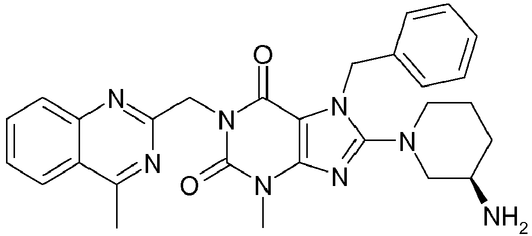
- 1-[(4-methyl-quinazolin-2-yl)methyl]-3-methyl-7-(2-butyn-1-yl)-8-(piperazin-1-yl)-xanthine (cf. WO 2005/051950, Example 1): 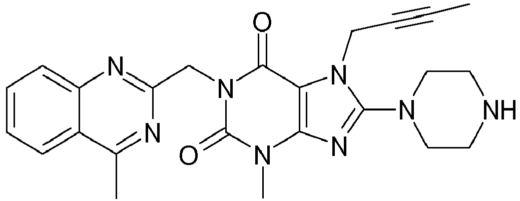
- 1-[(4-methyl-quinazolin-2-yl)methyl]-3-methyl-7-(2-butyn-1-yl)-8-(homopiperazin-1-yl)-xanthine (cf. WO 2005/051950, Example 1(3)): 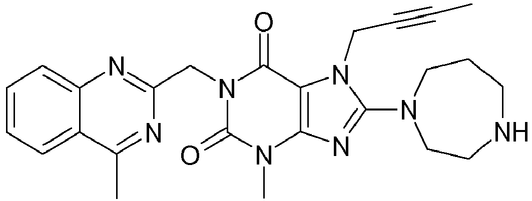
- 1-[(3-cyano-quinolin-2-yl)methyl]-3-methyl-7-(2-butyn-1-yl)-8-(3-(*R*)-amino-piperidin-1-yl)-xanthine (cf. WO 2005/085246, Example 1(30)): 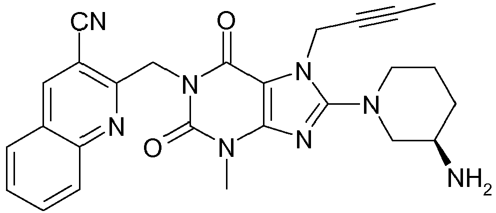
- 1-[(4-methyl-quinazolin-2-yl)methyl]-3-methyl-7-(2-butyn-1-yl)-8-[(*S*)-(2-aminopropyl)-methylamino]-xanthine (cf. WO 2006/029769, Example 2(4)): 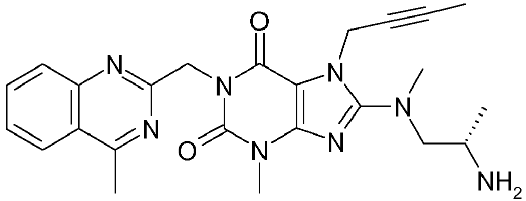
- 1-[(imidazo[1,2-a]quinolin-2-yl)methyl]-3-methyl-7-(2-butyn-1-yl)-8-(3-amino-piperidin-1-yl)-xanthine (cf. WO 2004/041820, Example 1(32)): 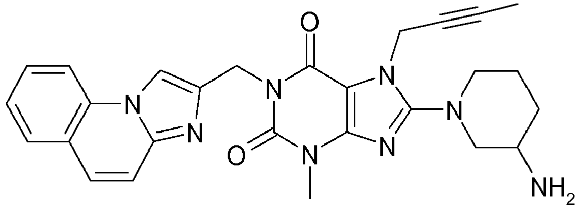
- 1-[(4-oxo-3,4-dihydro-quinazolin-2-yl)methyl]-3-methyl-7-(2-butyn-1-yl)-8-(3-amino-piperidin-1-yl)-xanthine (cf. WO 2004/018468, Example 2(71)): 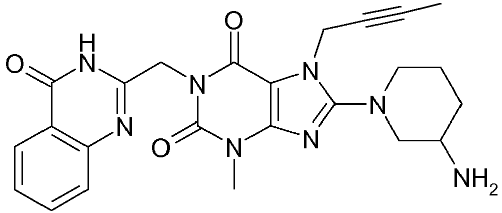
- 1-[(quinoxalin-2-yl)methyl]-3-methyl-7-(2-butyn-1-yl)-8-(3-amino-piperidin-1-yl)-xanthine (cf. WO 2004/018468, Example 2(169)): 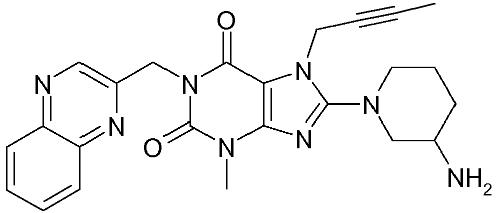
- 1-[(quinoxalin-6-yl)methyl]-3-methyl-7-(2-butyn-1-yl)-8-((*R*)-3-amino-piperidin-1-yl)-xanthine (cf. WO 2005/085246, Example 1(83)): 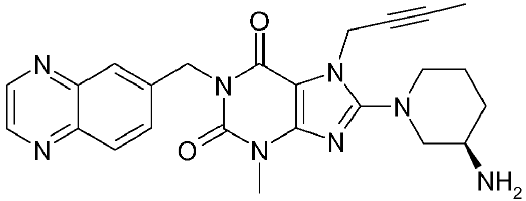
- 1-[(4-morpholino-quinazolin-2-yl)methyl]-3-methyl-7-(2-butyn-1-yl)-8-(3-amino-piperidin-1-yl)-xanthine (cf. WO 2004/018468, Example 2(180)): 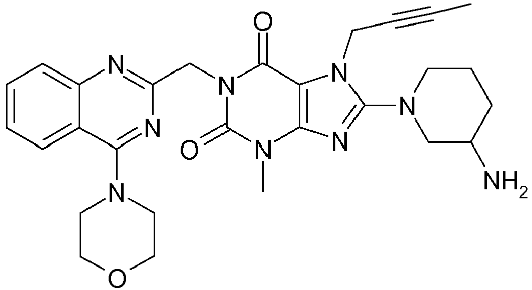
- 1-[(1-methyl-2-oxo-1,2-dihydro-quinolin-6-yl)methyl]-3-methyl-7-(2-butyn-1-yl)-8-(3-amino-piperidin-1-yl)-xanthine (cf. WO 2004/018468, Example 2(227)): 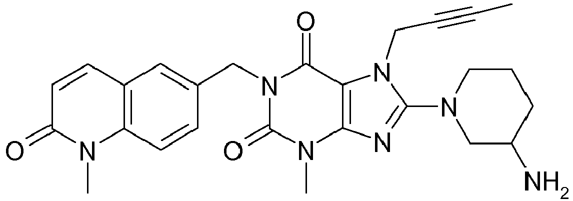
- 1-[(4-cyano-isoquinolin-3-yl)methyl]-3-methyl-7-(2-butyn-1-yl)-8-((*R*)-3-amino-piperidin-1-yl)-xanthine (cf. WO 2005/085246, Example 1(88)): 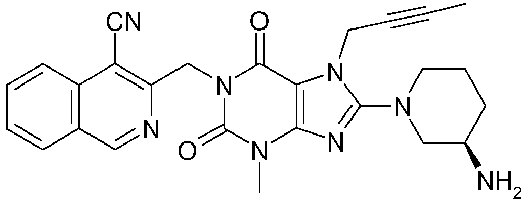
- 1-[(4-methyl-quinazolin-2-yl)methyl]-3-carboxymethyl-7-(2-butyn-1-yl)-8-(3-(*R*)-amino-piperidin-1-yl)-xanthine (cf. WO 2006/027204, Example 2): 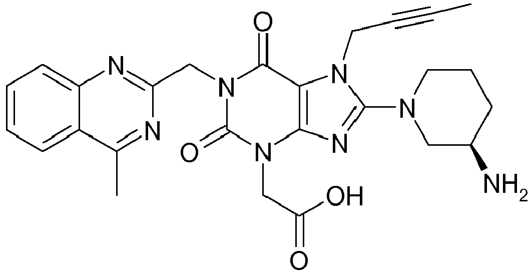
- 1-[(4,6-dimethyl-pyrimidin-2-yl)methyl]-3-methyl-7-(2-butyn-1-yl)-8-((*R*)-3-amino-piperidin-1-yl)-xanthine (cf. WO 2005/085246, Example 1(82)): 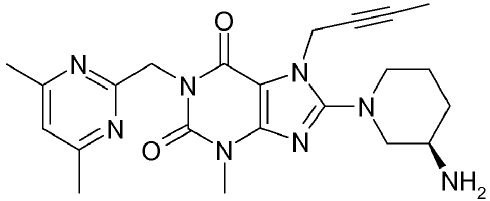
- 1-[(3-cyano-pyridin-2-yl)methyl]-3-methyl-7-(2-butyn-1-yl)-8-((*R*)-3-amino-piperidin-1-yl)-xanthine (cf. WO 2005/085246, Example 1(52)):

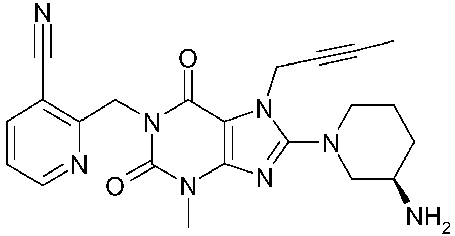

The biological properties of the compounds are investigated as follows:

### FAP Assay:

The FAP source used is a homogenised preparation of CD8huFAP cells and this is diluted with buffer in the ratio 1: 100. The substrate used for the reaction is H-AlaPro_7-amido-4-trifluoromethylcoumarin (AlaPro-AFC) made by Bachem (Prod. No I-1680).

The test substances are diluted in DMSO (dimethylsulphoxide) and buffer and pipetted into a 96-well plate. 70 uL of the enzyme and 20 uL of the substrate are added thereto. The plate is incubated for one hour at ambient temperature, then the fluorescence is measured using a Wallac Victor 1420 Multilabel Counter at an excitation wavelength of 405 nm and an emission wavelength of 535 nm.

The results were calculated by comparing the fluorescence in the presence of the test substance with the fluorescence of the control. The basal fluorescence was deducted.

The compounds according to the invention have an FAP inhibition of >50% at a concentration of 100 µM, for example. Preferably the compounds according to the invention have an FAP inhibition of >50% at a concentration of 3 µM.

For pharmaceutical use, the compounds according to the invention are generally used for warm-blooded vertebrates, particularly humans, in doses of 0.001-100 mg/kg of body weight, preferably 0.1-15 mg/kg, 1 to 4 times per day. For this purpose the compounds, optionally combined with another active substance, are formulated with one or more conventional inert carriers and/or diluents, e.g. with corn starch, lactose, glucose, microcrystalline cellulose, magnesium stearate, polyvinylpyrrolidone, citric acid, tartaric acid, water, water/ethanol, water/glycerol, water/sorbitol, water/polyethyleneglycol, propyleneglycol, cetylstearylalcohol, carboxymethylcellulose or fatty substances such as hard fat or suitable mixtures thereof to form conventional galenic preparations such as plain or coated tablets, capsules, powders, suspensions or suppositories.

The pharmaceutical preparation is designed in accordance with the desired method of administration (oral, intravenous, parenteral, etc), the preferred formulation being an oral preparation.

The pharmaceutical compositions according to the invention containing the above-mentioned purine derivatives are thus prepared by the skilled man using permitted formulation excipients by methods as described in the prior art. Examples of such excipients are diluents, binders, carriers, fillers, lubricants, flow agents, crystallisation retardants, disintegrants, solubilisers, colourings, pH regulators, surfactants and emulsifiers.

Examples of suitable diluents include cellulose powder, calcium hydrogen phosphate, erythritol, (low-substituted) hydroxypropylcellulose, mannitol, pregelatinised starch or xylitol.

Examples of suitable binders include copolymers of vinylpyrrolidone with other vinyl derivatives (copovidone), hydroxypropylmethylcellulose (HPMC), hydroxypropylcellulose (HPC) polyvinylpyrrolidone (povidone), pregelatinised starch, or low-substituted hydroxypropylcellulose.

Examples of suitable lubricants include talc, polyethyleneglycol, calcium behenate, calcium stearate, hydrogenated castor oil or magnesium stearate.

Examples of suitable disintegrants include maize starch or crospovidone.

Suitable methods of preparing pharmaceutical formulations of the DPP IV inhibitors according to the invention are
- Direct tabletting of the active substance in powder mixtures with suitable tabletting excipients;
- Granulation with suitable excipients and subsequent mixing with suitable excipients and subsequent tabletting as well as film coating; or
- packing of powder mixtures or granules into capsules.

Suitable granulation methods are
- wet granulation in the intensive mixer followed by fluidised bed drying;
- one-pot granulation;
- fluidised bed granulation; or
- dry granulation (e.g. by roller compaction) with suitable excipients and subsequent tabletting or packing into capsules.

As different functional disorders often occur simultaneously, it is not infrequently advisable to combine a number of different active principles with one another. Thus, depending on the functional disorders diagnosed, improved treatment outcomes may be obtained if an FAP inhibitor is combined with a (permitted) active substance commonly used for the ailment in question, e.g. with an active substance having anti-hyperproliferative properties or with an active substance that can be used for the treatment of hyperproliferative diseases (e.g. cancers).

Such a combined treatment may be given as a free combination of the active substances or in the form of a fixed combination, for example in a tablet or capsule. Pharmaceutical formulations of the combination partner needed for this may either be obtained commercially as pharmaceutical compositions or may be formulated by the skilled man using conventional methods. The active substances which may be obtained commercially as pharmaceutical compositions are described in numerous places in the prior art, for example in the list of drugs that appears annually, the "Rote Liste ®" of the Federal Association of the Pharmaceutical Industry, or in the annually updated compilation of manufacturers' information on prescription drugs known as the "Physicians' Desk Reference".

The anti-cancer treatment defined here may be used as a sole therapy or may comprise, in addition to the compound according to the invention, conventional surgery, radiation therapy or chemotherapy. Such chemotherapy may comprise one or more of the following categories of chemotherapeutic and/or targeted antitumour agents:

The following categories and some typical representatives of them may be mentioned as examples of known chemotherapeutic antitumour agents:
(i) alkylating/carbamylating active substances such as cyclophosphamide (Endoxan), Ifosfamide (Holoxan), thiotepa, melphalan (Alkeran) or chloroethylnitrosourea (CENU); (ii) platinum derivatives such as cisplatin (Platinex), oxaliplatin, satraplatin or carboplatin (Carboplat); (iii) antimitotic active substances / tubulin inhibitors such as vinca alkaloids (Vincristin, Vinblastin, Vinorelbin), taxanes such as paclitaxel (Taxol), docetaxel (Taxotere) or the analogues and conjugates thereof, epothilones such as epothilone B (Patupilone), azaepothilone (Ixabepilone) or ZK-EPO; (iv) topoisomerase inhibitors such as anthracycline (e.g. doxorubicin / Adriblastin), epipodophyllotoxins (e.g. etoposid / Etopophos) and camptothecin and camptothecin analogues (e.g. Irinotecan / Camptosar or Topotecan / Hycamtin); (v) pyrimidine antagonists such as 5-fluorouracil (5-FU), capecitabine (Xeloda), arabinosylcytosine / cytarabine (Alexan) or gemcitabine (Gemzar); (vi) purine antagonists such as 6-mercaptopurine (Puri-Nethol), 6-thioguanine or fludarabine (Fludara) and (vii) folic acid antagonists such as methotrexate (Farmitrexat) or premetrexed (Alimta).

The following categories and some typical representatives of them may be mentioned as examples of targeted antitumour agents used in experimental or standard cancer therapy:
(i) kinase (e.g. Abl, EGFR, VEGFR, PDGFR etc.) inhibitors such as imatinib (Glivec), ZD-1839 / fefitinib (Iressa), Bay43-9006 (Sorafenib, Nexavar), SU11248 / sunitinib (Sutent) or OSI-774 / erlotinib (Tarceva), dasatinib (Sprycel), ILapatinib (Tykerb), or vatalanib, vandetanib (Zactima) or pazopanib; (ii) proteasome inhibitors such as PS-341 / bortezumib (Velcade); (iii) heat shock protein 90 inhibitors such as 17-allylaminogeldanamycin (17-AAG); (iv) vascular targeting agents (VTAs) such as combretastin A4 Phosphat or AVE8062 / AC7700, as well as anti-angiogenic active substances such as VEGF antibodies such as bevacizumab (Avastin), angiokinase inhibitors or KDR tyrosinekinase inhibitors such as PTK787 / ZK222584 (Vatalanib) or vandetanib (Zactima) or pazopanib; (v) monoclonal antibodies such as trastuzumab (Herceptin), rituximab (MabThera / Rituxan), alemtuzumab (Campath), tositumomab (Bexxar), C225 / cetuximab (Erbitux), avastin or panitumumab, mutants and conjugates of monoclonal antibodies such as gemtuzumab ozogamicin (Mylotarg) or ibritumomab tiuxetan (Zevalin), as well as antibody fragments; (vi) oligonucleotide-based active substances such as G-3139 / oblimersen (Genasense); (vii) toll-like receptor / TLR 9 agonists such as ProMune, TLR 7 agonists such as imiquimod (Aldara) or isatoribine as well as analogues thereof, or TLR 7/8 agonists such as resiquimod, as well as immunostimulatory RNA as TLR 7/8 agonists; (viii) protease inhibitors (ix) hormonal active substances such as anti-oestrogens (such as tamoxifen or raloxifen), anti-androgens (such as flutamide or casodex), LHRH analogues (such as leuprolide, goserelin or triptorelin) as well as aromatase inhibitors.

The following active substances may be mentioned as examples of other targeted antitumour agents that may be useful in cancer therapy: bleomycin, retinoids such as all-trans retinoic acid (ATRA), DNA methyltransferase inhibitors such as decitabine (Docagen) or 5-azacytidine, alanosine, cytokines such as interleukin-2, interferons such as interferon-alpha2 or interferon-gamma, death receptor agonists such as TRAIL, DR4/5 agonistic antibodies, FasL and TNF-R agonists (e.g. TRAIL receptor agonists such as mapatumumab or lexatumumab), as well as HDAC inhibitors such as SAHA.

## Claims

1. Use of a compound
of formula (I) 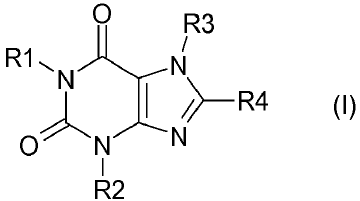
formula (II), 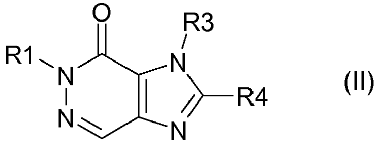
formula (III), 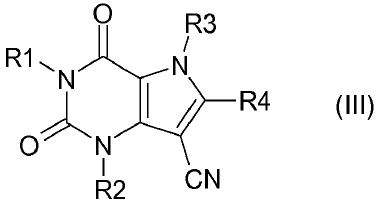
or formula (IV), 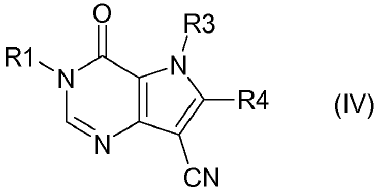 wherein
**R1** denotes pyridinylmethyl, pyrimidinylmethyl, quinolinylmethyl, (2-oxo-1,2-dihydro-quinolinyl)methyl, isoquinolinylmethyl, quinazolinylmethyl, (4-oxo-3,4-dihydro-quinazolinyl)methyl, quinoxalinylmethyl, [1,5]naphthyridinylmethyl, (1H-perimidinyl)methyl, phenanthridinylmethyl, (11H-dibenzo[b,e]azepinyl)methyl, (dibenzo[b,f][1,4]oxazepinyl)methyl, (5H-dibenzo[b,e][1,4]diazepinyl)methyl or (imidazo[1,2-a]quinolinyl)methyl and the heterocyclic groups of the above-mentioned groups may be mono- or disubstituted by Ra, while the substituents may be identical or different and **Ra** denotes fluorine, chlorine, bromine, cyano, methyl, ethyl, propyl, isopropyl, cyclopropyl, phenyl, methoxy, ethyloxy, amino, methylamino, dimethylamino, pyrrolidino, piperidino, morpholino, piperazino or N-methylpiperazino,
**R2** denotes methyl, ethyl, propyl, isopropyl, cyclopropyl or phenyl and the methyl, ethyl, propyl and isopropyl group may be substituted by carboxy, methoxycarbonyl, ethyloxycarbonyl, aminocarbonyl, methylaminocarbonyl, dimethylaminocarbonyl, pyrrolidinocarbonyl, piperidinocarbonyl, morpholinocarbonyl, piperazinocarbonyl or N-methylpiperazinocarbonyl,
**R3** denotes 2-buten-1-yl, 3-methyl-2-buten-1-yl, 2-butyn-1-yl, benzyl, fluorobenzyl, chlorobenzyl, bromobenzyl or cyanobenzyl and
**R4** denotes piperazino, homopiperazino, 3-(R)-amino-piperidin-1-yl, 3-(S)-amino-piperidin-1-yl, (2-amino-ethyl)-methylamino, (2-amino-2-methyl-propyl)-methylamino, ((R)-2-amino-propyl)-methylamino or ((S)-2-amino-propyl)-methylamino,
or one of the tautomers, enantiomers or salts thereof, or a mixture thereof,
for preparing a pharmaceutical composition for the treatment of diseases that respond to the inhibition of FAP.

2. The use according to claim 1, wherein said compound is defined formula I, II, III or IV, wherein
**R1** denotes pyridin-2-ylmethyl, pyridin-3-ylmethyl, pyridin-4-ylmethyl, pyrimidin-2-ylmethyl, pyrimidin-4-ylmethyl, quinolin-2-ylmethyl, quinolin-3-ylmethyl, quinolin-4-ylmethyl, quinolin-5-ylmethyl, quinolin-6-ylmethyl, quinolin-7-ylmethyl, quinolin-8-ylmethyl, (2-oxo-1,2-dihydro-quinolin-6-yl)methyl, isoquinolin-1-ylmethyl, isoquinolin-3-ylmethyl, isoquinolin-4-ylmethyl, isoquinolin-8-ylmethyl, quinazolin-2-ylmethyl, quinazolin-4-ylmethyl, quinazolin-6-ylmethyl, quinazolin-7-ylmethyl, (4-oxo-3,4-dihydro-quinazolin-2-yl)methyl, quinoxalin-2-ylmethyl, quinoxalin-5-ylmethyl, quinoxalin-6-ylmethyl, [1,5]naphthyridin-2-ylmethyl, [1,5]naphthyridin-3-ylmethyl, [1,5]naphthyridin-4-ylmethyl, (1H-perimidin-2-yl)methyl, phenanthridin-6-ylmethyl, (11H-dibenzo[b,e]azepin-6-yl)methyl, (dibenzo[b,f][1,4]oxazepin-11-yl)methyl, (5H-dibenzo[b,e][1,4]diazepin-11-yl)methyl or (imidazo[1,2-a]quinolin-2-yl)methyl, while the heterocyclic groups of the above-mentioned groups may be monosubstituted by cyano, ethyl, cyclopropyl, phenyl or morpholino or may be mono- or disubstituted by methyl,
or one of the tautomers, enantiomers or salts thereof, or a mixture thereof.

3. The use according to claim 1 or 2, wherein said compound is defined by formula I, II, III or IV, wherein
**R2** denotes methyl, carboxymethyl, methoxycarbonylmethyl, ethyloxycarbonylmethyl, cyclopropyl or phenyl,
or one of the tautomers, enantiomers or salts thereof, or a mixture thereof.

4. The use according to one of claims 1 to 3, wherein said compound is defined by formula I, II, III or IV, wherein
**R3** denotes 2-buten-1-yl, 3-methyl-2-buten-1-yl, 2-butyn-1-yl, benzyl, 2-chlorobenzyl, 2-bromobenzyl or 2-cyanobenzyl,
or one of the tautomers, enantiomers or salts thereof, or a mixture thereof.

5. The use according to one of claims 1 to 4, wherein said compound is defined by formula I, II, III or IV, wherein
**R4** denotes piperazino, homopiperazino, 3-(R)-amino-piperidin-1-yl, 3-(S)-amino-piperidin-1-yl, (2-amino-ethyl)-methylamino, ((R)-2-amino-propyl)-methylamino or ((S)-2-amino-propyl)-methylamino,
or one of the tautomers, enantiomers or salts thereof, or a mixture thereof.

6. The use according to one of claims 1 to 5, wherein said compound is defined by formula I or II.

7. The use according to one of claims 1 to 6, wherein said diseases that respond to the inhibition of FAP are hyperproliferative diseases.

8. The use according to one of claims 1 to 6, wherein said diseases that respond to the inhibition of FAP are cancers, such as for example tumour diseases of epithelial origin such as breast tumours, non-small-cell lung carcinomas, colorectal carcinomas or soft tissue carcinomas, and metastasising tumours such as melanomas.

9. The use according to one of claims 1 to 6, wherein said diseases that respond to the inhibition of FAP are hyperproliferative diseases other than cancers, such as for example cardiac hypertrophy, cirrhoses, fibromatoses, rheumatoid arthritis, osteoarthritis, neurotraumatic diseases, pain, migraine, wound healing disorders, acne, proliferative skin diseases, such as e.g. Psoriasis.

10. Process for preparing a pharmaceutical composition for the treatment of a disease as defined in one of claims 1, 7, 8 and 9, **characterised in that** a compound as defined in one of claims 1 to 6 is used.

11. A compound as defined in one of claims 1 to 6 for use in the treatment of a disease as defined in one of claims 1, 7, 8 and 9.
